# DEMANDE DE BREVET EUROPEEN

(11) **EP 3 851 086 A1**
(43) Date de publication de la demande: **21.07.2021**
(21) Numéro de dépôt: 21150257.0
(22) Date de dépôt: 05.01.2021
(51) Int. Cl.: A61H 1/00, A61F 2/00, A61H 39/04, A61F 5/24, A61F 5/28, A61F 13/00, A61F 5/455

(54) **DISPOSITIF DE MAINTIEN EXTERNE DE L'URÈTRE FEMININ À L'EFFORT**

(30) Priorité: 17.01.2020 FR 2000430; 17.11.2020 FR 2011794
(71) Demandeur: Eurl Cornier, 75116 Paris (FR)
(72) Inventeur: CORNIER, EDGARD, 75116 PARIS (FR)
(74) Mandataire: Cabinet HERRBURGER

(57) **Abrégé**

Dispositif de soutien directionnel externe de l'urètre féminin à l'effort, constitué par un coussin (1) rigide fixé à un contre-appui rigide (2) placé dans la partie postérieure d'une ceinture ou d'une culotte, et comportant une partie de support périnéale, le contre-appui rigide (2) étant relié en avant par des lanières réglables (2) le soutiennent à une ceinture (5) ou une culotte dont la périphérie arrière est rigidifiée par un insert ou autre matériau ne pliant pas.

## Description

### DOMAINE DE L'INVENTION

La présente invention a pour objet un dispositif de maintien externe de l'urètre féminin à l'effort.

Selon un nouveau postulat(http://perineologie-vectorielle.com) développé par l'inventeur, les forces abdominales ne se dirigent pas verticalement vers le plancher pelvien. Elles sont réfléchies par la surface de l'ensemble osseux et musculaire du petit bassin. Ces forces sont physiquement redirigées vers l'avant. Les frottements des tissus relies entre eux sont retenus par le tissus conjonctifs jeunes. (reference A2499EC European Journal of Obstetrics & Gynecology and Reproductive Biology 234 (2019) - From tissue viscoelasticity to vectorial perineology - Ed- gard Cornier1,2,*, et al.) les pressions produisent des mouvements quasi statiques de plicature des organes entre eux. La Plicature des organes créent des zones appelées « fonctionnelles ». Ce sont ces zones qui font obstacles aux fuites. L'invention consiste à corriger ces renvois de forces afin de recréer la plicature fonctionnelle des organes du petit bassin. Un appui postérieur permettra avec la redirection de ces vecteurs de force, la flexion de l'urètre féminin, sa clôture, et la guérison des incontinences urinaires à l'effort. Le reste du pelvis, au centre, et latéralement (les grandes lèvres et les tissus adjacents) ne doivent pas être maintenus et il doivent rester libre de leurs mouvements.

### ETAT DE LA TECHNIQUE

L'incontinence urinaire affecte environ 20% des femmes jeunes et 77% à l'age du relâchement tissulaire (Urinary Incontinence in Women: A Review.
Lukacz ES, Santiago-Lastra Y, Albo ME, Brubaker L.JAMA. 2017 Oct 24;318(16):1592-1604.)

L'arsenal thérapeutique est pauvre et la plupart des femmes affectées sont réduites au port de couches absorbantes.

La rééducation musculaire à elle seule est peu efficace dans la durée, ou contraignante. Il faut lui adjoindre différentes méthodes, qui supposent un investissement personnel difficile à assumer sur le long terme. L'arrêt de l'entrainement s'accompagne de récidives.

Kegel Exercises, Biofeedback, Electrostimulation, and Peripheral Neuro-modulation Improve Clinical Symptoms of Fecal Incontinence and Affect Specific Physiological Targets: An Randomized Controlled Trial. Mundet L, Rofes L, Ortega O, Cabib C, Clavé P.J Neurogastroenterol Motil. 2020 Oct 28.

La mise en place de pessaires intra vaginaux est inconfortable, peu suivie, peu efficaces sur les fuites urinaires. Pessary use in stress urinary incontinence: a review of advantages, complications, patient satisfaction, and quality of life.
Al-Shaikh G, Syed S, Osman S, Bogis A, Al-Badr A.Int J Womens Health. 2018
Souvent il a été fait appel à la chirurgie pour mettre en place une bandelette de soutien direct sous urétral pour provoquer la flexion forcée de l'urètre, pour sa fermeture à l'effort. Cette intervention chirurgicale a connu une grande diffusion tant la gêne est importante. Mais le pourcentage élevé de complications en a maintenant interdit ou très fortement limités l'usage. (Survey on surgery for stress urinary incontinence in an era mid-urethral slings are being questioned. D'hulster AS, Housmans S, Spaans W, Van der Aa F, Slabbaert K, Milani AL, Deprest J.Int Urogynecol J. 2020 Apr;31(4):695-702.).

Les dispositifs de soutien de l'ensemble du périnée selon l'arrière-plan technologique, on connaît les documents suivants : EP 2 158 882, US 2015/034074, JP 2014 1510-20. Ces brevets antérieurs proposent des dispositif placés sur tout le périnée et maintenus par des bretelles. Leur but est d'empêcher les descentes des prolapsus, et les refoulant largement, à l'aide d'un dispositif convexe ou concave, vers l'intérieur de l'orifice vaginal.

Ces dispositifs sont plus ou moins larges et prennent appui à l'extérieur de la vulve, sur les grandes lèvres. Ils comportent parfois une gouttière en avant pour permettre aux urines de s'échapper. Aucun ne revendique le traitement des fuites urinaires de la femme.

Ces dispositifs soulèvent, l'ensemble des organes du petit bassin très au-delà de la zone postérieure du périnée, et soulèvent la totalité des tissus pelviens. Ils sont destinés à maitriser les grandes descentes d'organe. Ils n'ont pas d'effet de plicature - clôture de l'urètre qu'ils soulèvent et rendent rectilignes. De ce fait ils sont mêmes susceptibles d'aggraver « de novo » les fuites urinaires. (reference : Commentary on 'De novo urinary incontinence after pelvic organ prolapse surgery-a national database study'.
Abdelrahman A.Int Urogynecol J. 2020 Feb;31(2):309.)

### BUT DE L'INVENTION

La présente invention a pour but de développer un dispositif de soutien remédiant aux inconvénients des solutions connues et de mettre la plicature de l'urètre lors d'un effort abdominal.

### EXPOSE ET AVANTAGES DE L'INVENTION

A cet effet, l'invention a pour objet un dispositif de soutien directionnel externe de l'urètre féminin à l'effort, caractérisé en ce qu'il comprend
- un coussin rectangulaire rigide muni d'une lanière postérieure et de deux lanières antérieures et
- une ceinture abdominale munie d'un point de fixation arrière et de deux points de fixation avant recevant de manière réglable respectivement la lanière postérieure et les deux lanières avant pour tenir le coussin rigide contre la zone postérieure et inférieure du périnée en partant du coccyx.

Le dispositif de soutien directionnel selon l'invention permet de réorienter les forces de pression abdominale, lors de l'effort abdominal, grâce à la mise en place postérieure de ce contre-appui rigide, plat et étroit, placé en avant du coccyx, ne recouvrant pas l'ensemble de la surface périnéale et restant à distance de l'orifice urinaire. Il permettra la plicature occlusive de la zone fonctionnelle de l 'urètre.

Suivant une caractéristique, la ceinture a un renforcement postérieur portant le point de fixation arrière.

Suivant une autre caractéristique, la ceinture est combinée à une culotte et notamment le coussin rigide est intégré dans la partie intermédiaire de la culotte reliant la partie avant et la partie arrière.

La culotte a une partie arrière renforcée au niveau de la ceinture portant le point de fixation arrière et une partie arrière rigide reliée au renforcement de la partie interfessiaire de la culotte.

Suivant une autre caractéristique avantageuse, la culotte a une partie avant munie d'une double paroi formant un canal de passage pour chacune des lanières avant pour rejoindre les deux points de fixation réglables sur le côté antérieur.

De façon avantageuse, les points de fixation réglables antérieurs sont constitués chacun par un double anneau surmontant l'orifice de sortie de la lanière correspondante. Cette réalisation permet une fixation réglable facile et simple à mettre en œuvre.

De façon avantageuse, le point de fixation réglable arrière comprend une boucle de passage fixée au renforcement postérieur et la lanière comporte une extrémité dentée d'enclipsage permettant d'accrocher la lanière à l'une des dents de la boucle pour réaliser un réglage fixe.

Ce réglage une fois effectué reste maintenu alors que le réglage au niveau des points de fixation avant reste réglable et peut être resserré ou desserré à tout moment en fonction de nécessité ou de la sensation de confort.

De façon particulièrement avantageuse, le coussin rigide est intégré dans la région inférieure postérieure de la partie intermédiaire de la culotte, l'avant du coussin étant muni des deux lanières passant dans les deux canaux de la double paroi avant pour rejoindre les deux points de fixation avant.

Ce mode de réalisation est particulièrement avantageux car pratique et confortable.

Les dimensions moyennes du coussin rigide sont de l'ordre de : longueur de 5 cm à 11 cm et largeur de 2 centimètres.

Une enveloppe souple formée par une protection biocompatible entoure la partie rigide, constituée par la plaquette

La plaquette rigide porte un dispositif ou un anneau à chacune de ses extrémités permettant de le relier à la lanière postérieure et aux deux lanières antérieures. Ces lanières sont reliées à une ceinture simple ou combinée/intégrée à une culotte.

La ceinture ou la culotte comportent un insert ou un matériau rigide dans leur partie arrière.

En résumé, la ceinture formant un bandeau ou la culotte se placent sans douleur au-dessus du relief fessier au niveau des ailes iliaques. Le bandeau ou la culotte sont renforcés dans leur tiers postérieur par un insert qui permet au bandeau ou à la ceinture de la culotte de ne pas fléchir à la tension.

Ce moyen permet d'obtenir un maintien confortable à l'ensemble.

### BREVE PRESENTATION DES DESSINS

La présente invention sera décrite de manière détaillée à l'aide de mode de réalisation de dispositifs de soutien selon l'invention représentés dans les dessins annexés dans lesquels :
[Fig. 1] une vue en plan schématique du coussin rigide.
[Fig. 2A] vue de face de l'ensemble du dispositif de coussin mis en place selon un premier mode de réalisation,
[Fig. 2B] vue arrière
[Fig. 3] vue schématique du coussin rectangulaire et de la ceinture au niveau du point de fixation arrière
[Fig. 4A] vue de détail d'un mode de réalisation de la fixation arrière
[Fig. 4B] vue de détail de deux points de fixation avant selon un mode de réalisation de l'invention
[Fig. 5] vue en perspective d'un premier mode de réalisation d'une culotte
[Fig. 6A] vue de face d'un autre mode de réalisation d'une culotte avec un coussin intégré
[Fig. 6B] vue de la partie intermédiaire de la culotte de la figure 6A
[Fig. 6C] vue arrière de la culotte de la figure 6A
[Fig. 6D] vue de la double paroi avant
[Fig. 7] schéma anatomique du petit bassin soulignant le renvoi vectoriel des forces exercées dans le petit bassin sur le périnée
[Fig. 8] schéma anatomique montrant le renvoi complémentaire des forces par un dispositif de maintien selon l'invention placé sous l'arrière du périnée.

### DESCRIPTION DE MODES DE REALISATION

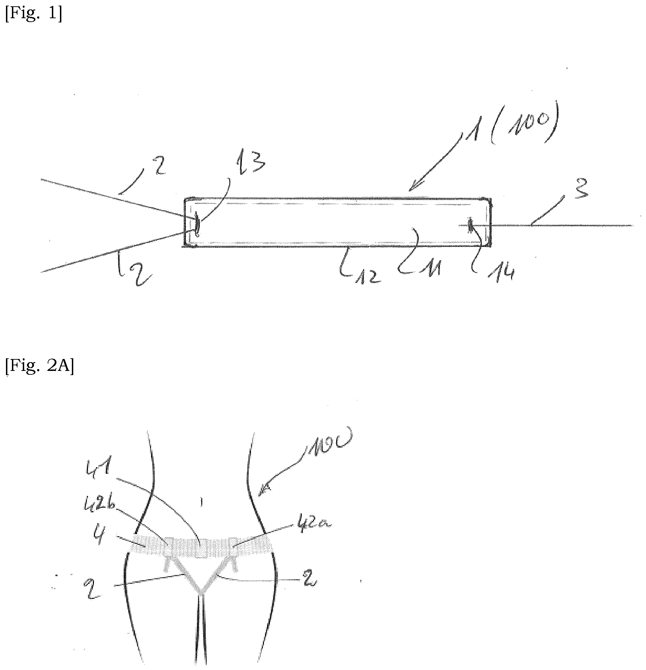

La vue de dessus schématique de la figure 1 montre un coussin 1 selon l'invention composé d'une plaquette rigide 11 logée dans une enveloppe souple 12. La plaquette est munie d'une attache avant 13 et d'une attache arrière 14. L'attache arrière 14 reçoit une lanière postérieure 2 et l'attache avant reçoit deux lanières avant 3.

La plaquette rigide a une longueur de l'ordre de 5 à 11cm et une largeur de l'ordre de 2cm. L'enveloppe souple 12 qui habille la plaquette rigide est une enveloppe libre ou une enveloppe fixée ou intégrée à la plaquette 11.

Selon un mode de réalisation, le coussin 1 est réalisé par bi-injection en deux matières plastiques, une matière relativement rigide formant la plaquette 11 et une matière souple biocompatible formant l'enveloppe 12.

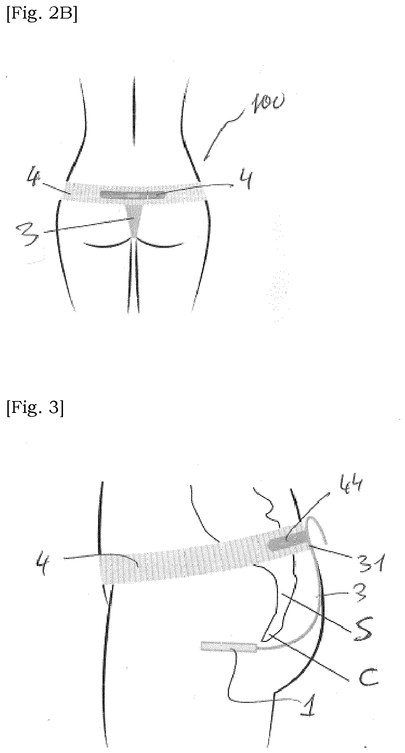

Le dispositif de soutien directionnel externe 100 selon l'invention se compose du coussin 1 tel que représenté à la figure 1 et d'une ceinture 4 comme celle présentée aux figures 2A et 2B.

La ceinture 4 est une ceinture large, élastique, munie le cas échéant d'une fermeture 41 permettant de régler la ceinture. A l'avant la ceinture 4 est munie de deux points de fixation 42a, 42b et à l'arrière d'un point de fixation arrière 43 fixé à un renfort arrière 44.

La figure 2A montre les deux lanières antérieures 2, reliées respectivement à l'un et l'autre des points de fixation 42a, 42b en divergeant en forme de V.

La figure 2B montre la lanière postérieure 3, reliée au point de fixation arrière 43. Les lanières avant sont de préférence élastiques et remontent vers la ceinture en s'écartant le long des plis de l'aine pour passer à travers la ceinture 4. Elles permettent le réglage de la tension à tout moment pendant le port du dispositif de soutien 100.

La ceinture 4 se place sans douleur au-dessus du relief fessier au niveau des ailes iliaque. Le bandeau formé par la ceinture 4 est renforcé dans son tiers postérieur par un renfort 44 qui permet à la ceinture de ne pas fléchir à la tension pour un maintien confortable de l'ensemble du dispositif de soutien 100.

Le schéma anatomique de la figure 3 montre le positionnement de la ceinture 4 avec son renfort arrière 44 et le point de fixation arrière représenté schématiquement. Le coussin 1 est installé en avant du coccyx C.

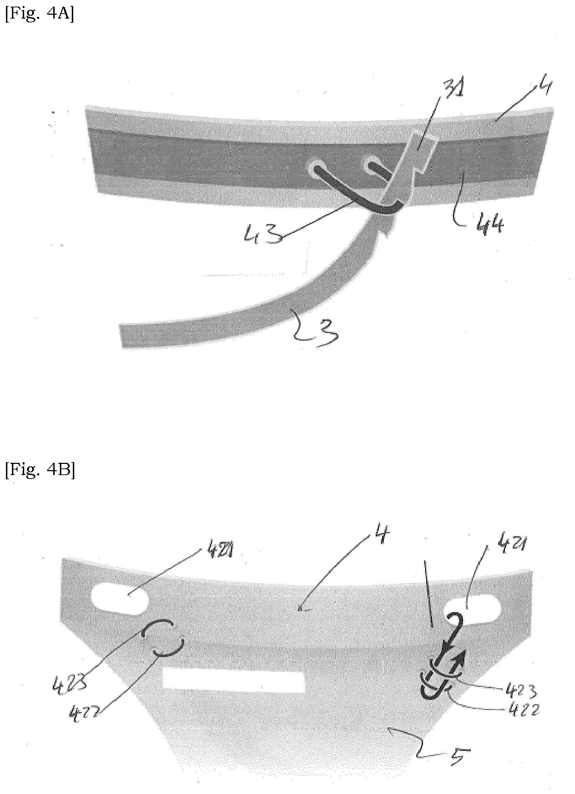

La figure 4A montre un mode de réalisation du point de fixation arrière 43 et de la lanière postérieure 3, munie de dents formant une sorte de crémaillère permettant l'accrochage de manière réglable dans la boucle constituée par le point de fixation arrière 43 relié au renfort 44 de la ceinture 4. Le réglage arrière se fait une fois la ceinture 4 mise en place. Le dispositif de réglage est constitué par la combinaison de l'anneau 43 du renfort 44 et de la partie dentée à l'extrémité de la lanière postérieure 3. Ce réglage une fois effectué reste maintenu fixe. Ainsi l'extrémité arrière du coussin 1 est mis en place définitivement contre le coccyx C.

La figure 4B montre un mode de réalisation des deux points de fixation avant 42a, 42b de la ceinture 4 représentée ici intégrée à une culotte 5.

La ceinture comporte deux orifices 421 de passage des lanières antérieures 2 à droite et à gauche et au niveau de ces orifices, deux anneaux couplés 422, 423 permettant le passage en S de la lanière antérieure 2 à travers le premier anneau 422 puis autour du second anneau 423 de façon que les deux boucles de la lanière 2 soient bloquées entre les deux anneaux 422, 423 par la traction de la lanière 2 reliée à l'extrémité avant du coussin 1.

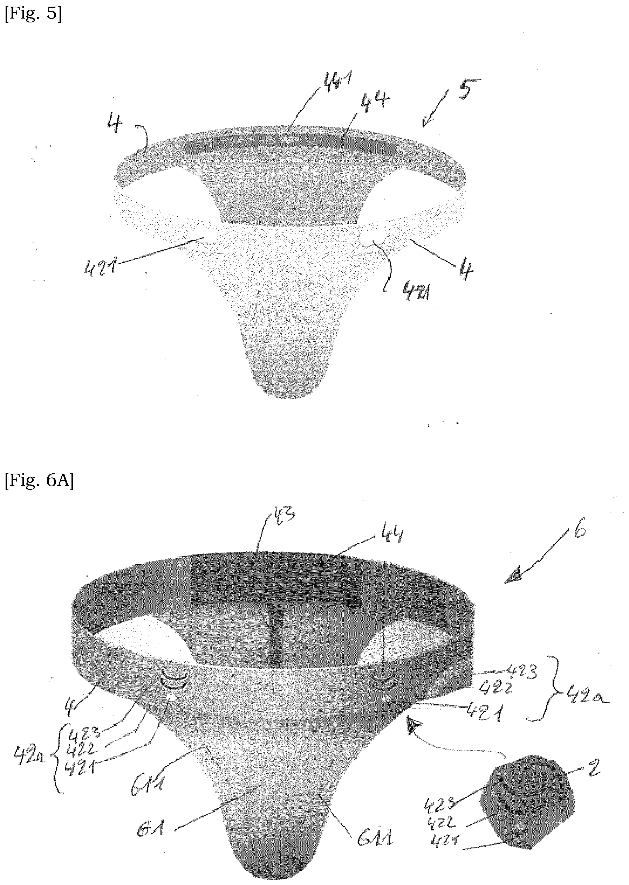

La figure 5 est une vue en perspective très schématique d'une ceinture 4 intégrée à une culotte 5. La ceinture 4 a la structure décrit ci-dessus avec deux orifices 421 à l'avant pour le passage des lanières antérieures 2 et à l'arrière un renfort 44 muni d'une ouverture 441 pour le passage de la lanière arrière 3. Cette ouverture 441 peut être réalisée dans le renfort 4ou dans la partie arrière de la culotte en-dessous de la ceinture 4.

Le point de fixation arrière 43 extérieur à la ceinture 4 n'est pas visible à la figure 5.

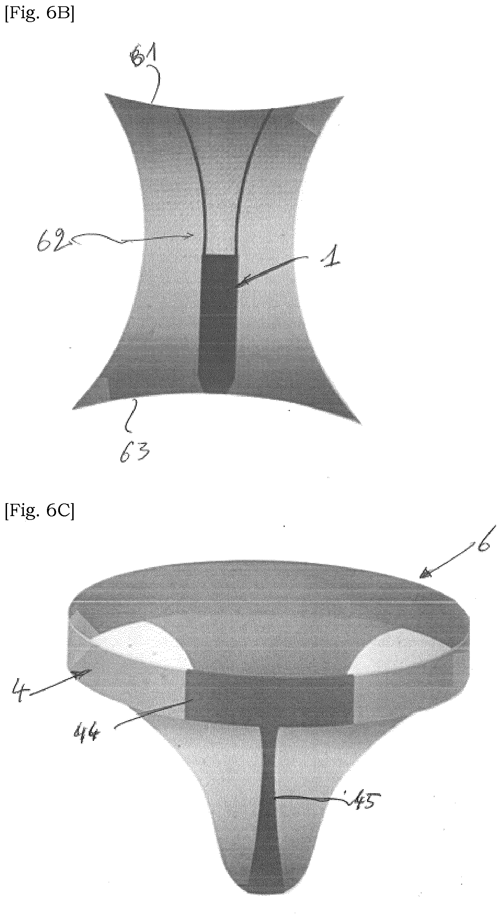

Les figures 6A, 6B, 6C montrent un autre mode de réalisation d'une culotte 6 intégrant le coussin 1 dans la culotte et formant ainsi par combinaison, le dispositif de soutien directionnel externe 100 selon l'invention.

La figure 6A est une vue du devant 61 en perspective de la culotte 6 avec la ceinture 4 correspondant aux modes de réalisation décrits ci-dessus.

La figure 6B montre l'intégration du coussin 1 dans la partie intermédiaire 62 de la culotte 6. Le coussin 1 porte les deux lanières antérieures 2. A l'arrière (fig. 6C) la culotte 6 a un renfort intégré dans la partie arrière 63 de la culotte et qui est relié à la ceinture 4 et à son renfort 44 et son prolongement 45.

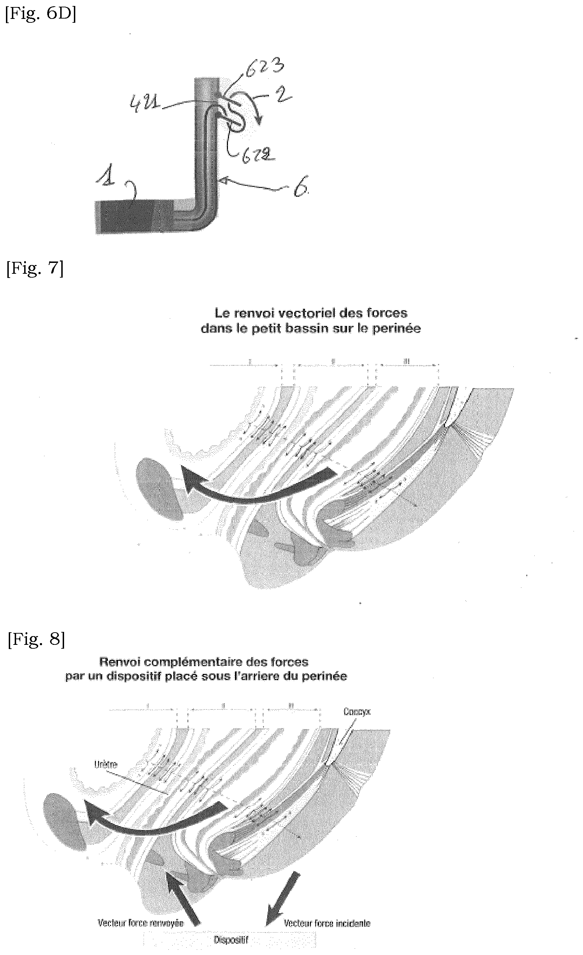

Les deux lanières antérieures 2 passent dans la double paroi avant de la culotte dans deux canaux 611 débouchant sous ou au niveau de la ceinture 4, près des deux anneaux 42a,b. Les lanières antérieures 2 non représentées à la figure 6A sortent pas les deux orifices 421 pour entourer les deux boucles 422, 423 de chaque point de fixation avant 42a,b. (voir aussi Fig. 6D).

La figure 7 montre par une flèche courbe, le renvoi vectoriel des forces dans le petit bassin sur le périnée.

La figure 8 montre très schématiquement l'effet du dispositif de maintien 100, pour renvoyer le vecteur force incidente sous la force d'un vecteur force renvoyé complétant le renvoi vectoriel de la figure 7.

De façon plus détaillée, l'effort abdominal entraîne un glissement des organes entre eux (Flèche rouge). Les organes sont élastiques et retenus entre eux par le tissu conjonctif souple (forces de frottement). Ces mouvements retenus par les ligaments (pubo urétraux), provoquent la plicature des tissus les uns sur les autres, à la manière de la plicature du tuyau d'arrosage du jardinier. Cette plicature arrête l'écoulement, on l'appelle zone fonctionnelle de l'urètre.

En cas de relâchement des tissus conjonctifs, ou arrachement des ligaments pubo urétraux, rien ne retient plus le tissu entre eux, et la plicature se fait mal (incontinence des urines par hypermobilité urétro-vesicale).

L'invention permet de réorienter les forces afin que cette plicature puisse se réaliser, (fig.8)

Elle consiste en une plaque rigide et étroite.

Cette plaque est ajustable contre la partie dure du petit bassin (le coccyx) en arrière.

Sa position sur l'axe de rotation postérieur est réglable pour réorienter plus ou moins haut les vecteurs des forces réfléchies.

Sa longueur ne dépasse pas les deux tiers postérieurs du périnée, afin de conserver la mobilité indépendante du tiers antérieur. Sa largeur ne dépasse pas la fente vaginale entre les petites lèvres, afin de conserver la mobilité indépendante des parties latérales du périnée.

Cette plaque rigide courte et étroite, ajustable et réglable agit afin de réorienter la direction des frottements dus à l'écoulement quasi statique des pressions abdominales. Une plicature peut se reproduire, et la zone fonctionnelle et l'urètre (appelée aussi sphincter) peut devenir active.

### NOMENCLATURE DES ELEMENTS PRINCIPAUX

100 Dispositif de soutien directionnel externe
1 Coussin
11 Plaquette rigide
12 Enveloppe
13 Attache avant
14 Attache arrière
2 Lanières antérieures
3 Lanière postérieure
31 Extrémité de la lanière postérieure
4 Ceinture
41 Fermeture
42a,b Point de fixation avant/double anneau
421 Orifice
422, 423 Anneaux couplés
43 Point de fixation arrière, anneau
44 Renfort
45 Prolongement de renfort arrière
441 Ouverture
5 Culotte combinée à un dispositif de maintien
6 Culotte intégrant le dispositif de maintien

## Revendications

1. Dispositif de soutien directionnel externe (100) de l'urètre féminin à l'effort abdominal,
**caractérisé en ce qu'**il comprend
- un coussin (1) rectangulaire, rigide, muni d'une lanière postérieure (3) et de deux lanières antérieures (2) et
- une ceinture abdominale (4) munie d'un point de fixation arrière (43) et de deux points de fixation avant (42a,b) recevant de manière réglable respectivement la lanière postérieure (3) et les deux lanières antérieures (2) pour tenir le coussin rigide (1) contre la zone postérieure et inférieure du périnée en partant du coccyx.

2. Dispositif de soutien directionnel (100) selon la revendication 1,
**caractérisé en ce que**
la ceinture (4) a un renfort postérieur (4) portant le point de fixation arrière (43).

3. Dispositif de soutien directionnel (100) selon la revendication 1,
**caractérisé en ce que**
la ceinture (4) est combinée à une culotte (6).

4. Dispositif de soutien directionnel (100) selon la revendication 3,
**caractérisé en ce que**
le coussin (1) rigide est intégré dans la partie intermédiaire (62) de la culotte (6) reliant la partie avant (61) et la partie arrière (63).

5. Dispositif de soutien directionnel (100) selon la revendication 4,
**caractérisé en ce que**
la culotte (6) a une partie arrière (63) renforcée au niveau de la ceinture (4) portant le point de fixation arrière (43) et le fond de culotte présente une partie arrière rigide reliée au renforcement de la liaison interfessiaire de la culotte.

6. Dispositif de soutien directionnel (100) selon la revendication 4,
**caractérisé en ce que**
la culotte (6) a une partie avant (61) munie d'une double paroi formant un canal de passage pour chacune des lanières antérieures pour rejoindre côté extérieur les deux points de fixation réglables.

7. Dispositif de soutien directionnel selon les revendications 1 et 6,
**caractérisé en ce que**
les points de fixation réglables extérieurs (42a,b) avant sont constitués chacun par deux anneaux couplés (422, 423) surmontant l'orifice de sortie (421) de la lanière antérieure (2) correspondant.

8. Dispositif de soutien directionnel selon les revendications 1 et 6,
**caractérisé en ce que**
le point de fixation arrière (43) réglable comprend une boucle de passage fixée au renforcement postérieur (44) et la lanière (3) comporte une extrémité (31) dentée d'enclipsage permettant d'accrocher la lanière (3) par l'une de ses dents à la boucle pour fixer le réglage.

9. Dispositif de soutien directionnel selon les revendications 4 à 6,
**caractérisé en ce que**
le coussin (1) rigide est intégré dans la région inférieure postérieure de la partie intermédiaire (62) de la culotte (6), l'avant du coussin (1) étant muni de deux lanières antérieures (2) passant dans les deux canaux de la double paroi avant pour rejoindre les deux points de fixation avant (42a,b).
